# FASCICULE DE BREVET EUROPEEN

(11) **EP 0 591 053 B1**
(45) Date de publication et mention de la délivrance du brevet: **29.01.1997**
(21) Numéro de dépôt: 93402381.3
(22) Date de dépôt: 29.09.1993
(51) Int. Cl.: A61N 1/05

(54) **Electrode pour appareil de stimulation cardiaque à vis biologique rétractable**
Herzschrittmacher-elektrode mit biologischer Stellschraube
Electrode for heart stimulator comprising a biological retractable screw

(30) Priorité: 01.10.1992 FR 9211786
(43) Date de publication de la demande: 06.04.1994
(73) Titulaire: SOCIETE ETUDES ET DEVELOPPEMENTS S.E.D., F-19130 Objat (FR)
(72) Inventeur: Jammet, Jean, Firmin, F-19130 Objat (FR)
(74) Mandataire: Thébault, Jean-Louis

(56) Documents cités:
- EP-A- 0 015 229
- DE-A- 3 712 082
- FR-A- 2 464 079

## Description

La présente invention a pour objet une électrode pour appareil de stimulation cardiaque à vis biologique rétractable.

Les appareils de stimulation cardiaque comprennent, de façon connue, un boitier extérieur muni d'une source d'énergie et d'un système électronique de commande, relié au moyen d'une liaison par câble à une électrode, elle-même fixée à l'intérieur du coeur, directement sur la paroi interne de l'une des cavités concernées.

Le problème est d'introduire une telle électrode par l'un des vaisseaux jusque dans la cavité concernée puis d'accrocher cette électrode sur la paroi, cet accrochage devant être suffisamment efficace pour permettre la transmission de signaux électriques.

**On connaît un premier document, la demande DE-A-3 712 082 qui décrit une sonde cardiaque avec une hélice d'accrochage qui peut être rentrée ou sortie d'une tête d'électrode par vissage. Des éléments de manoeuvre permettent de bloquer en rotation l'hélice tout en autorisant les mouvements de translation.**

De nombreux **autres** dispositifs ont été développés et on connaît plus particulièrement celui qui est décrit dans le brevet francais FR-A-2 575 925 concernant une électrode de stimulation et de détection pour sonde cardiaque.

Afin de réaliser l'ancrage de cette électrode, il est prévu de disposer une vis biologique rétractable dans un corps cylindrique, constituant l'électrode proprement dite.

Un câble conducteur, souple et solidaire du corps, permet de déplacer ce corps constituant l'électrode jusque dans la cavité cardiaque concernée, la vis biologique étant en position escamotée, à l'intérieur du corps durant tout ce déplacement.

La vis biologique est en réalité montée sur un élément mobile en translation à l'intérieur du corps, cet élément mobile étant une vis mécanique montée tournante dans un taraudage ménagé à l'intérieur du corps de l'électrode.

Cette vis mécanique comprend à sa partie supérieure un trou borgne destiné à recevoir un outil de manoeuvre qui n'est autre qu'un second câble muni d'un embout prévu pour coopérer avec le trou borgne de la vis et monté libre, coaxialement et à l'intérieur du câble conducteur.

Le fonctionnement et la mise en oeuvre de cette électrode sont les suivants :
- introduction de l'électrode avec la vis biologique en position rétractée à l'intérieur du vaisseau jusque dans la cavité concernée,
- lorsque l'extrémité libre de l'électrode vient en butée contre la paroi de la cavité, manoeuvre en rotation du câble intérieur au câble conducteur par le praticien, et
- mise en rotation de la vis mécanique par le câble conducteur, ce qui a pour effet de déplacer en translation la vis biologique d'ancrage hors du corps de l'électrode.

Lorsque l'électrode est en appui contre la paroi concernée, cette vis biologique , dont la pointe est effilée, pénètre dans le tissu fibreux du muscle cardiaque.

Il suffit ensuite de relier l'extrémité libre du câble conducteur avec l'appareil de stimulation proprement dit.

Le contact électrique avec les tissus est bien assuré, ce qui diminue la consommation électrique nécessaire et conduit à des résultats de transmission de signaux plus efficaces.

Dans cette demande de brevet FR-A-2 575 925, il est également prévu des moyens d'ancrage par des appendices souples répartis sur la périphérie extérieure du corps de l'électrode.

Si ce type de montage donne toute satisfaction quant à la qualité de l'ancrage et à celle du contact électrique il subsiste néanmoins un problème concernant la mise en oeuvre de cette électrode.

En effet, le praticien doit maintenir l'électrode en pression contre la paroi de la cavité avant de manoeuvrer la vis biologique.

Deux cas peuvent se présenter, soit l'électrode est bien en contact avec la paroi, et la vis biologique simultanément à sa mise en saillie, pénètre et s'accroche dans le tissu de la paroi, soit l'électrode est légèrement en retrait de la paroi de la cavité, contrairement à ce que suppose le praticien.

Dans ce dernier cas, la vis biologique est mise en rotation, simultanément à la vis mécanique, ce qui provoque la mise en saillie de la vis biologique mais celle-ci ne s'accroche pas dans la paroi.

L'ancrage peut être indûment considéré comme réalisé par le praticien.

De plus, la sensibilité tactile du praticien est faussée par le fait que, entre la vis biologique et l'extrémité libre du câble de manoeuvre, il est prévu l'ensemble vis mécanique-taraudage qui ne transmet pas fidèlement les réactions de la vis biologique.

Il faut également constater que l'électrode ayant un diamètre de l'ordre de 3 mm, il est difficile de doser le couple en utilisant le montage qui vient d'être décrit.

Compte tenu de l'encombrement nécessairement très faible de l'électrode, la longueur de la vis mécanique est réduite, ce qui oblige, dans le cas où l'électrode n'est pas ancrée, à mettre en rotation dans le sens inverse le câble de manoeuvre pour que la vis mécanique recule et dispose à nouveau d'une course suffisante pour autoriser la pénétration complète de la vis biologique lors de sa mise en saillie.

Aussi, la présente invention pallie cet inconvénient en proposant une électrode à vis biologique rétractable qui permet au praticien de "ressentir" l'accrochage et l'ancrage de la vis biologique dans la paroi et cette électrode permet en outre de manoeuvrer la vis biologique en rotation jusqu'à ce qu'elle pénètre dans la paroi sans aucune limitation du nombre de tours et en effectuant une manoeuvre en rotation dans un seul sens jusqu'à l'ancrage.

Une variante selon l'invention prévoit aussi la mise en rotation du corps de la vis.

L'électrode selon la présente invention peut également être retirée par rotation inverse lorsque la nécessité s'en fait sentir.

D'autre part, cette électrode, tout en assurant un excellent contact électrique, évite en outre toute détérioration de la partie mécanique par pénétration de liquide à l'intérieur de l'électrode, ceci grâce à un joint fixe d'une grande efficacité, autour de la vis biologique.

A cet effet, l'électrode selon la présente invention, du type à vis biologique rétractable et prévue pour un appareil de stimulation cardiaque, comprend un corps muni d'un filetage interne, une vis mécanique interne coopérant avec ce filetage et une vis biologique solidaire de cette vis mécanique par une de ses extrémités, l'autre extrémité, de forme hélicoïdale, étant prévue pour pénétrer dans les tissus fibreux du coeur par rotation et cette électrode se caractérise en ce que la vis mécanique est munie d'une gorge "point mort" disposée en partie amont du filetage.

Cette électrode se caractérise également en ce qu'elle comprend un joint fixe, monté dans la partie aval du corps et bloqué par des moyens de plaquage dans celui-ci par une rondelle.

Selon une autre caractéristique, les moyens de plaquage comprennent une seconde rondelle en amont bloquée en appui contre un suralésage du corps de l'électrode qui reçoit l'une des extrémités d'un ressort de rappel interposé entre la vis mécanique et cette rondelle.

Selon une variante les moyens de plaquage comprennent une première rondelle fixe par rapport au corps immédiatement en amont du joint, une seconde rondelle en appui sur l'extrémité amont du joint et un second ressort, coaxial au premier, passant à travers la première rondelle et venant en appui sur cette seconde rondelle, les deux ressorts venant en appui par leur extrémité libre sur la vis mécanique.

Selon un mode de réalisation préférentiel de l'invention, la vis mécanique comprend un fourreau intérieur monté coaxial, faisant saillie à l'extrémité amont de ladite vis mécanique, l'extrémité aval de ce fourreau ou le prolongement de la vis mécanique permettant le centrage du ou des ressorts, tandis que la partie amont du fourreau est prévue pour coopérer avec un outil de manoeuvre.

Selon une caractéristique particulière, l'extrémité amont de la vis biologique est bloquée à l'intérieur du fourreau par réalisation d'un méplat, sur le fourreau et sur la vis biologique.

De même l'extrémité libre amont du fourreau, prévue pour recevoir l'extrémité libre de l'outil de manoeuvre, est également aplatie.

Un câble conducteur du type ressort à spires jointives est solidarisé à l'extrémité amont du corps de l'électrode, l'ensemble du corps, du câble étant protégé extérieurement par une gaine.

Selon un mode de réalisation particulier, la vis mécanique est munie d'un filetage qui coopère avec un ergot fixe en saillie à l'intérieur du corps de l'électrode.

Cet ergot est en l'occurrence un axe incliné disposé sensiblement suivant une corde de la cavité cylindrique interne du corps de l'électrode.

Selon une variante, cet ergot est une déformation du corps même de l'électrode.

La présente invention est décrite ci-après selon un mode de réalisation particulier et une variante en regard des dessins annexés sur lesquels :
- la figure 1 représente une vue en perspective de l'électrode selon l'invention et d'une portion du câble conducteur et du câble de manoeuvre,
- la figure 2 représente une vue en coupe longitudinale de l'électrode selon l'invention,
- la figure 3, représente une vue en coupe longitudinale de l'électrode selon l'invention mais avec la vis biologique en saillie,
- les figures 4, 5 et 6 représentent des vues en coupe transversale respectivement suivant les lignes 4-4, 5-5, 6-6 de la figure 2,
- les figures 7A et 7B représentent un premier synoptique de mise en oeuvre de l'électrode selon l'invention,
- les figures 8A, 8B, 8C représentent un second synoptique de mise en oeuvre de l'électrode selon l'invention,
- la figure 9 représente une variante de réalisation de l'électrode et plus particulièrement des moyens de plaquage du joint et de l'ergot de la vis mécanique,
- la figure 10 est identique à la figure 9 mais avec la vis biologique en saillie, et
- les figures 11A, 11B, 11C et 11D représentent le synoptique de mise en oeuvre de cette variante de réalisation.

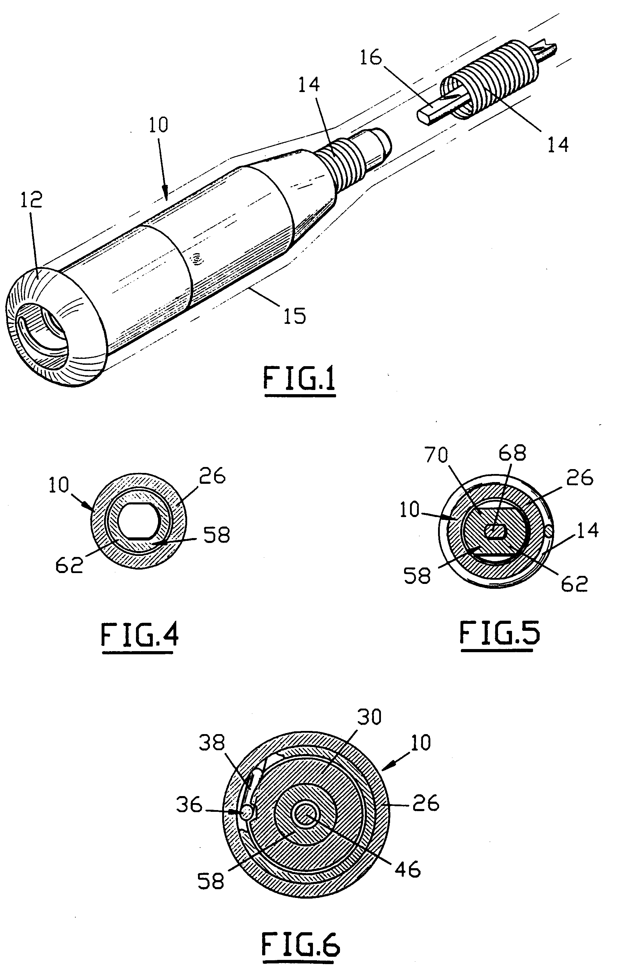

Sur la figure 1, on a représenté le corps 10 de l'électrode, muni d'une lèvre 12, de forme arrondie à son extrémité aval, et d'un câble conducteur 14 solidaire de son extrémité amont, recouvert d'une gaine continue 15.

Ce câble conducteur 14 est relié, de façon connue, à l'appareil de stimulation cardiaque proprement dit (non représenté).

Sur cette même figure 1, on a partiellement représenté le câble de manoeuvre 16, muni de méplats.

L'extrémité aval de l'électrode est ouverte et l'on peut voir l'extrémité effilée de la dernière spire de la vis biologique, représentée dans sa position rétractée et qui sera décrite en détail ci-après.

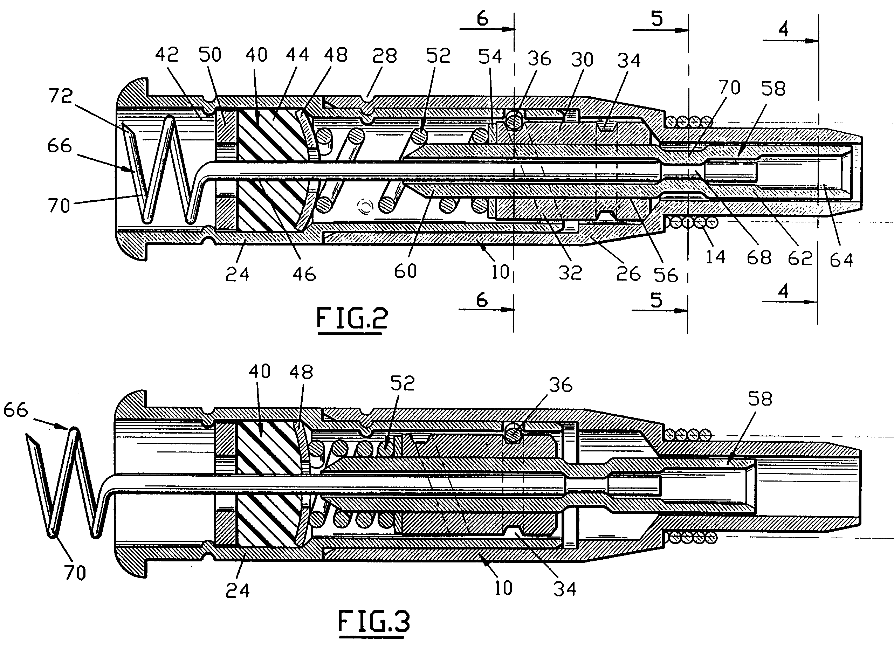

Sur la figure 2, le corps 10 de l'électrode est réalisé en deux parties, l'une 24 formant le demi-corps aval et l'autre 26 formant le demi-corps amont, ces deux demi-corps étant liés mécaniquement par des poinçonnages 28 répartis à 120° dans l'exemple représenté.

Le câble conducteur 14, à spires jointives, est disposé à l'extrémité libre du demi-corps amont 26.

A l'intérieur du corps 10 de l'électrode, il est prévu une vis mécanique 30 munie d'un filetage 32 et d'une gorge "point mort" 34, disposée en amont du filetage 32.

Ce filetage et cette gorge sont prévus pour coopérer avec un ergot 36 en saillie à l'intérieur du corps 10 de l'électrode.

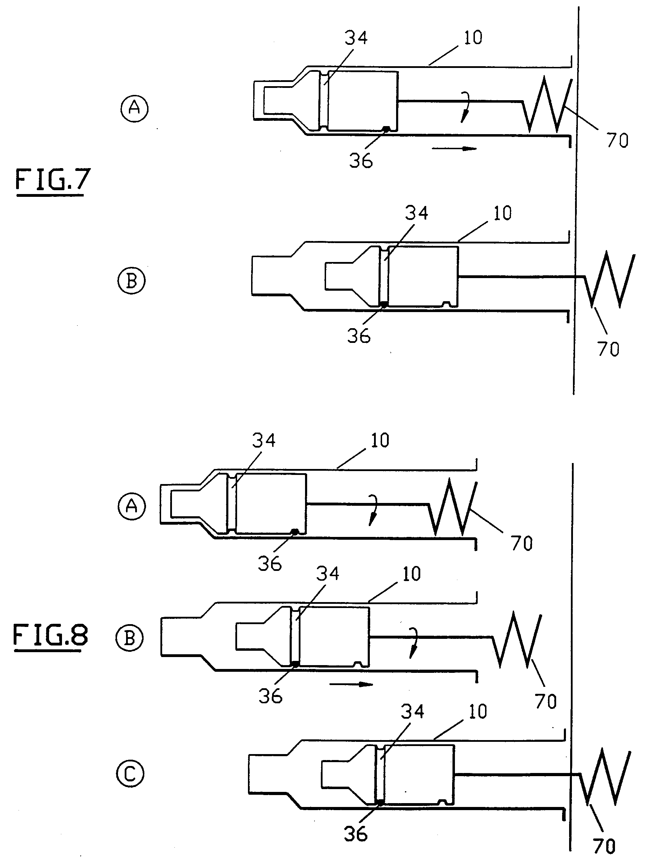

Cet ergot 36 est mieux représenté sur la coupe de la figure 6 et il comprend un axe cylindrique 38, incliné et disposé suivant une corde de la cavité cylindrique intérieure du corps de l'électrode.

Cette électrode comprend en outre un joint fixe 40, disposé dans un suralésage 42 ménagé à l'intérieur du corps de l'électrode à proximité de la partie aval.

Ce joint 40 comprend une bague 44 en élastomère munie d'un trou 46, sans enlèvement de matière, centré suivant l'axe longitudinal du corps de l'électrode ainsi que deux rondelles amont 48 et aval 50. La rondelle 50 aval est montée dans le suralésage 42 afin de bloquer la bague 44, et elle est immobilisée par la déformation de la paroi, grâce à des poinçonnages, tandis que la rondelle 48, en butée sur le fond du suralésage, assure le blocage en position de cette bague en élastomère.

Par ailleurs, on constate que cette rondelle est légèrement incurvée vers l'amont sous l'effet de la compression du joint par la rondelle 50.

Entre la vis mécanique 30 et cette rondelle 48, on interpose un ressort 52 dont l'extrémité amont vient en appui sur une rondelle 54 plaquée contre l'extrémité aval de la vis mécanique 30.

Cette vis mécanique 30 comprend également un alésage 56, centré, à l'intérieur duquel un fourreau 58 est monté en force.

Ce fourreau fait saillie à l'extrémité aval de la vis par une portion 60 et le ressort 52 comprimé fait office de butée.

Ce fourreau fait également saillie en amont par une portion 62 amont. Cette partie amont du fourreau comprend un suralésage 64 débouchant vers l'amont.

L'électrode selon l'invention, représentée sur cette figure 2, comprend également une vis biologique 66 munie à son extrémité amont d'un étranglement 68 bloqué dans une partie aplatie 70 du fourreau 58, et comprenant à son extrémité aval une hélice 70 dont l'extrémité libre 72 est effilée.

Sur la figure 2, la vis biologique est en position rétractée et la partie axiale traverse le fourreau 58, le ressort 52, la rondelle amont 48, la bague en élastomère 44 et la rondelle de blocage 50, l'hélice 70 étant entièrement contenue dans le suralésage 42.

Sur la figure 3, on a représenté une vue sur laquelle les références concernant les éléments identiques sont conservées, mais dans ce cas la vis mécanique 30 est en position translatée, telle que l'ergot 36 est dans la gorge "point mort" 34, le ressort 52 est en position comprimée, la rondelle 48 est légèrement aplatie et l'hélice 70 est en saillie à l'extérieur du corps de l'électrode.

Sur les figures 4 et 5, on a représenté des coupes transversales et les éléments identiques portent les mêmes références que ceux des figures 2 et 3.

Le fonctionnement et la mise en oeuvre de l'électrode qui vient d'être décrite, sont détaillés ci-après.

La mise en place de l'électrode selon l'invention, effectuée par le praticien, consiste à introduire dans un des vaisseaux le corps de l'électrode 10 et à le pousser grâce au câble conducteur 14 jusque dans la cavité du coeur concernée, les figures afférentes sont les figures 7A et 7B.

Lorsque le praticien estime, grâce à ses moyens médicaux de contrôle, que l'électrode est au contact de la paroi sur laquelle elle doit être ancrée, il introduit le câble de manoeuvre 16 à l'intérieur du câble conducteur 14 jusqu'à ce qu'il pénètre dans le suralésage 64, la vis biologique 66 étant en position rétractée.

Il peut donc manoeuvrer en translation ce câble 16 sans aucun risque de déplacement de la vis biologique.

Il suffit alors au praticien de faire tourner le câble de manoeuvre pour que la vis mécanique 30, qui est solidaire du fourreau 58, soit entraînée en rotation et que le filetage 32 coopère avec l'ergot 36, fixe par rapport au corps de l'électrode.

La mise en rotation de cette vis mécanique 30, provoque son déplacement à l'intérieur du corps de l'électrode jusqu'à ce que l'ergot 36 coopère avec la gorge "point mort" 34.

La vis biologique de l'électrode est alors en saillie dans la position représentée sur la figure 3.

Comme l'électrode était au contact de la paroi interne de la cavité concernée du coeur, la vis biologique, grâce à sa pointe effilée a pénétré dans le tissu fibreux.

Le praticien fait tourner le câble de manoeuvre jusqu'à sentir une résistance, témoin d'un bon ancrage de la vis biologique. La transmission du couple résistant est très bonne puisqu'elle est directe.

Le nombre de tours est illimité puisque la vis mécanique coopère avec l'ergot en saillie 36 par l'intermédiaire de sa gorge "point mort" qui autorise la rotation sans provoquer de déplacement.

La vis biologique, ainsi que l'électrode, sont dans la position représentée sur la figure 7B, le trait vertical représentant la paroi de tissu fibreux.

Dans certains cas, le praticien peut estimer à tort que l'électrode est en contact avec la paroi de la cavité, notamment à cause de l'imprécision des moyens de contrôle et de visualisation.

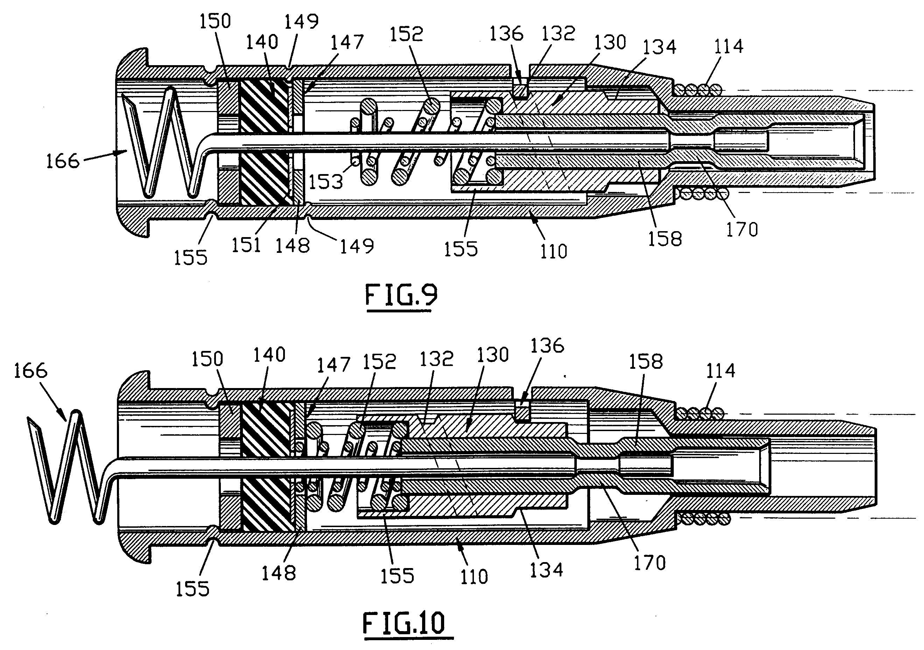

Dans ce cas, l'électrode est dans la position représentée sur la figure 8A, c'est-à-dire que la vis biologique est rétractée et l'extrémité aval de l'électrode est légèrement éloignée de la paroi de la cavité.

Ainsi qu'indiqué précédemment, le praticien introduit le câble de manoeuvre et met en saillie la vis biologique, l'électrode se trouve alors dans la position représentée sur la figure 8B.

On constate que la vis a tourné dans le vide et n'a pas pénétré dans le tissu fibreux de la cavité.

Aussi, le praticien ne "sent" aucun couple résistant bien que plusieurs tours de vis aient été effectués. Informé de ce fait directement par la manipulation, il lui suffit alors de faire effectuer une translation supplémentaire au câble conducteur pour avancer l'électrode, tout en faisant effectuer à la vis biologique des rotations grâce au câble de manoeuvre.

L'électrode se rapproche de la cavité jusqu'à ce que la vis biologique "morde" dans le tissu fibreux. Le praticien va ressentir une résistance dès que l'extrémité aval du corps de l'électrode viendra en appui sur le tissu fibreux et que la vis biologique aura totalement pénétré. L'ancrage est alors réalisé.

L'électrode selon l'invention a donc cet avantage important de permettre un ancrage en une seule opération simple même lorsque le corps de l'électrode n'est pas en contact direct avec la paroi sur laquelle elle doit être placée.

La manipulation est simple et le nombre de pièces constituant l'électrode est réduit ce qui la rend d'autant plus fiable.

En ce qui concerne la qualité électrique des contacts, elle est de bonne qualité compte tenu du fait que l'étanchéité du joint fixe autour de la vis biologique est renforcée lorsque cette vis biologique est translatée puisque le ressort exerce une pression sur la rondelle amont 48 qui vient comprimer la bague 44 en élastomère autour de cette vis biologique, ce qui évite toute détérioration des composants conducteurs.

D'autre part, le joint est prévu d'un diamètre légèrement supérieur à celui du suralésage 42 afin d'améliorer l'étanchéité à la façon d'un bouchon de bouteille.

Le ressort a également une autre fonction qui est celle d'exercer un effort permanent sur la vis mécanique, si bien que l'électrode selon l'invention peut être retirée aisément par rotation inverse du câble de manoeuvre qui entraîne la vis mécanique en rotation inverse puisque dès que l'ergot 36 en saillie croise le filetage, il y pénètre.

Sur les figures 9 et 10, on a représenté un autre mode de réalisation dont la mise en oeuvre peut avoir la préférence de praticiens.

Les références identiques des figures 2 et 3 sont les mêmes augmentées de 100 pour les figures 9 et 10.

Ce mode de réalisation comprend un corps 110 unique dans lequel est montée une vis mécanique 130 à laquelle est solidarisée une vis biologique 166, par l'intermédiaire d'un fourreau 158.

Une rondelle fixe 150 est bloquée par rapport au corps 110 par des déformations 155 régulièrement réparties à la périphérie.

Un joint d'étanchéité 140, disposé en amont de cette rondelle vient en appui sur cette rondelle et, en périphérie, sur l'intérieur du corps 110.

En amont du joint, il est prévu des moyens de plaquage 147 comprenant une première rondelle 148 fixe par rapport au corps grâce à des déformations 149 et une seconde rondelle 151 disposée libre entre l'extrémité amont du joint et la première rondelle.

La rondelle fixe 150, le joint 140 et les première et seconde rondelles 148 et 151 des moyens de plaquage sont munis d'un trou central à travers lequel passe la vis biologique.

Le trou sans enlèvement de matière à travers le joint 140 est ajusté sur le corps de la vis biologique.

Le trou central de la première rondelle est d'un diamètre supérieur à celui du trou de la seconde rondelle.

Les moyens de plaquage comprennent également un ressort 153 solidaire de la vis mécanique 130, d'une longueur telle qu'il n'exerce au repos, c'est-à-dire lorsque la vis mécanique est en retrait, aucun effort sur la seconde rondelle libre 151.

Le ressort 153 a un diamètre compris entre les diamètres des première et seconde rondelles 148 et 151.

La vis mécanique 130 comprend de la même façon que dans le mode de réalisation principal un ressort de rappel 152.

Les deux ressorts 152 et 153 sont coaxiaux et guidés par une jupe 155 qui prolonge l'extrémité aval de la vis mécanique 130.

La vis mécanique 130 est inchangée, elle comprend un filetage 132 et une gorge "point mort" 134.

L'ergot 136 est réalisé par déformation d'une partie du corps de l'électrode. Deux découpes transversales sur une portion d'arc libère une lame qui est déformée vers l'intérieur du corps pour venir en saillie à l'intérieur du corps formant l'ergot.

La gorge peut être également ouverte vers l'amont, comme représenté sur les figures 9 et 10.

Cet ergot a des dimensions prévues pour coopérer avec le filetage 132.

Les autres éléments de l'électrode sont inchangés. La vis biologique est fixée à la vis mécanique par un étranglement 170 et le câble conducteur 114 reste solidaire de l'extrémité amont du corps 110.

Le fonctionnement et la mise en oeuvre de cette variante sont quelque peu différents de celui de l'électrode précédente.

En effet, l'électrode est amenée de la même façon dans la cavité concernée du coeur.

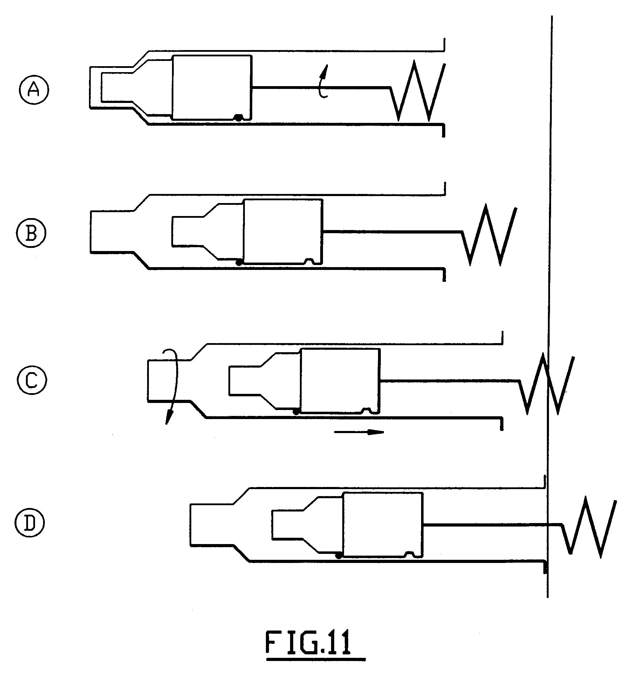

C'est l'étape A de la figure 11, au cours de laquelle, un câble de manoeuvre prévu pour coopérer en rotation avec la vis mécanique, permet par action de rotation de la part du praticien, de mettre le vis biologique en saillie, le corps étant maintenu fixe en rotation.

En variante, le câble de manoeuvre peut rester fixe et le praticien peut tourner le corps de l'électrode, le résultat étant identique, à savoir faire sortir la vis biologique.

On remarque que cette étape a lieu avant que le corps ne vienne au contact de la paroi de la cavité.

Durant l'étape B, le praticien retire le premier câble de manoeuvre pour le remplacer par un second câble de manoeuvre à bout rond qui interdit toute action en rotation sur la vis mécanique.

Ce câble de manoeuvre, courbé par le praticien, autorise la mise en place de l'électrode en des endroits plus difficilement accessibles.

Le praticien pousse alors le corps de l'électrode avec le câble de manoeuvre qui appuie sur la vis mécanique tout en tournant le corps 110 de l'électrode.

Les forces de frottement de la vis dans le joint, des ressorts et de la vis mécanique avec l'ergot sont suffisantes pour assurer la pénétration par vissage de la vis biologique dans le tissu cardiaque.

A l'étape C, la vis biologique pénètre dans le tissu.

Lorsque la vis biologique est entièrement vissée, le praticien est informé qu'il peut arrêter quant il ressent la rotation du corps par rapport à la vis mécanique.

En effet, les forces de frottement sont insuffisantes par rapport à l'effort exercé par le praticien puisque la vis biologique est bloquée en rotation dans le tissu.

La gorge "point mort" évite tout déplacement relatif du corps par rapport à la vis mécanique et le praticien ressent un "cliquetis" lorsque l'ergot "saute", le filetage pour rester dans la gorge "point mort". C'est l'étape D.

Durant toutes ces étapes, et dès que la vis biologique est en saillie, le ressort 153, passant à travers la première rondelle 148, exerce son effort sur la seconde rondelle 151, poussé par la vis mécanique 130.

La rondelle fixe 150 permet à la seconde rondelle 151 de comprimer le joint 140, ce qui assure une amélioration de l'étanchéité première due à un surdimensionnement du joint d'une part autour de la vis biologique et d'autre part sur la paroi intérieure du corps 110.

La gaine continue 15, extérieure et qui recouvre l'ensemble du corps et du câble conducteur, assure l'étanchéité périphérique.

Le raccordement du câble conducteur est effectué de façon connue sur l'appareillage de stimulation proprement dit.

## Revendications

1. Electrode à vis biologique rétractable prévue pour un appareil de stimulation cardiaque, comprenant un corps (10,110) creux, une vis mécanique (30, 130) interne coopérant **par** un filetage (32, 132) **avec un ergot (36, 136) solidaire** du corps et une vis biologique (66, 166) solidaire de cette vis mécanique par une de ses extrémités, l'autre extrémité, de forme hélicoïdale, étant prévue pour pénétrer dans les tissus fibreux du coeur par rotation, caractérisée en ce que la vis mécanique (30, 130) est munie d'une gorge "point mort" (34, 134) disposée en partie amont du filetage **(32, 132)** de la vis mécanique, **de façon à permettre une rotation de la vis mécanique par rapport au corps ou inversement, sans provoquer de déplacement supplémentaire de la vis biologique par rapport au corps dès que l'ergot se situe dans ladite gorge "point mort".**

2. Electrode selon la revendication 1, caractérisée en ce qu'elle comprend un joint (40, 140) fixe, comportant une bague (44, 144) en élastomère montée dans la partie aval du corps (10, 110) de cette électrode et bloqué en force dans celui-ci par une rondelle (50).

3. Electrode selon la revendication 1 ou 2, caractérisée en ce que le joint (40) comprend en amont des rondelles (48, 148, 151) bloquées en appui contre le fond d'un suralésage (42) du corps (10) de l'électrode.

4. Electrode selon la revendication 3, caractérisée en ce qu'il est prévu de disposer des ressorts (52, 152, 153) entre l'extrémité aval de la vis mécanique (30, 130) et les rondelles (48, 148, 151) disposées en amont du joint (40, 140) fixe.

5. Electrode selon l'une quelconque des revendications précédentes, caractérisée en ce que la vis mécanique (30, 130) comprend un fourreau (58, 158) intérieur monté coaxial faisant saillie à l'extrémité amont de ladite vis, l'extrémité aval de ce fourreau ou le prolongement de la vis mécanique permettant le centrage des ressorts (52, 152, 153), tandis que la partie amont du fourreau est prévue pour coopérer avec un câble de manoeuvre.

6. Electrode selon la revendication 1 et la revendication 5, caractérisée en ce que l'extrémité amont de la vis biologique comprend un étranglement de blocage (68, 168) à l'intérieur du fourreau (58, 158) par réalisation d'un méplat (70, 170).

7. Electrode selon l'une quelconque des revendications précédentes, caractérisée en ce que l'extrémité libre amont du fourreau (58, 158), prévue pour recevoir l'extrémité libre du câble de manoeuvre, est également aplatie.

8. Electrode selon l'une quelconque des revendications précédentes, caractérisée en ce qu'un câble conducteur (14, 114) du type ressort à spires jointives est solidarisé à l'extrémité amont du corps de l'électrode.

9. Electrode selon l'une quelconque des revendications précédentes, caractérisée en ce que la vis intérieure (30, 130) est munie d'un filetage (32, 132) et coopère avec un ergot (36, 136) en saillie à l'intérieur du corps de l'électrode qui constitue le filetage intérieur.

10. Electrode selon l'une quelconque des revendications précédentes, caractérisée en ce que l'ergot (36, 136) est un axe incliné (38) et disposé sensiblement suivant une corde de la cavité cylindrique interne du corps (10) de l'électrode, ou une déformation (136) venue du corps (110) lui-même.

## Claims

1. Electrode with retractable biological screw designed for use in a cardiac stimulation device, comprising a hollow body (10, 110), an internal mechanical screw (30, 130) cooperating by means of a thread (32, 132) with a pin (36, 136) which is joined to the body and a biological screw (66, 166) which is joined to the mechanical screw by one of its ends, the other end, which is in helicoidal form, being designed to penetrate the fibrous tissues of the heart by rotation, characterized in that the mechanical screw (30, 130) is equipped with a "dead centre" groove (34, 134) disposed upstream of the thread (32, 132) of the mechanical screw, in such a way as to allow the mechanical screw to rotate in relation to the body, or vice versa, without causing any additional displacement of the biological screw in relation to the body once the pin is inserted into the said "dead centre" groove.

2. Electrode as claimed in claim 1, characterized in that it comprises a fixed joint (40, 140) formed from an elastomer ring (44, 144) mounted in the downstream part of the body (10, 110) of the electrode and tightly locked on this by means of a washer.

3. Electrode as claimed in claim 1 or 2, characterized in that the upstream part of the joint (40) comprises washers (48, 148, 151) which are locked against the bottom of a bore (42) in the body (10) of the electrode.

4. Electrode as claimed in claim 3, characterized in that it is equipped with springs (52, 152, 153) between the downstream end of the mechanical screw (30, 130) and the washers (48, 148, 151) disposed upstream of the fixed joint (40, 140).

5. Electrode as claimed any of the previous claims, characterized in that the mechanical screw (30, 130) comprises a coaxial mounted internal sleeve (58, 158) which extends into the upstream end of the said screw, the downstream end of the said sleeve or extension of the mechanical screw allowing the springs (52, 152, 153) to be centred, whilst the upstream part of the sleeve is designed to cooperate with a control cable.

6. Electrode as claimed in claim 1 and claim 5, characterized in that the upstream end of the biological screw comprises a locking neck (68, 168) inside the sleeve (58, 158) formed by flattening (70, 170).

7. Electrode as claimed in any of the previous claims, characterized in that the free upstream end of the sleeve (58, 158) is designed to receive the free end of the control cable is also flattened.

8. Electrode as claimed in any of the previous claims, characterized in that a conduction cable (14, 114) of the type consisting of a spring with contiguous spirals is joined to the upstream end of the body of the electrode.

9. Electrode as claimed in any of the previous claims, characterized in that the internal screw (30, 130) is equipped with a thread (32, 132) and cooperates with a pin (36, 136) which extends into the body of the electrode which forms the internal thread.

10. Electrode as claimed in any of the previous claims, characterized in that the pin (36, 136) is an inclined axis (38) and is roughly disposed along a line of the internal cylindrical cavity of the body (10) of the electrode, or a deformation (136) coming from the body (110) itself.

## Patentansprüche

1. Herzschrittmacherelektrode mit biologischer Stellschraube, umfassend einen hohlen Körper (10, 110), eine innere mechanische Schraube (30, 130), die über ein Gewinde (32, 132) mit einer fest mit dem Körper verbundenen Nase (36, 136) zusammenarbeitet, und eine biologische Schraube (66, 166), die mit einem ihrer Enden an dieser mechanischen Schraube befestigt ist, während das andere Ende von spiraliger Form vorgesehen ist, um durch Drehen in die faserigen Herzgewebe einzudringen, dadurch gekennzeichnet, daß die mechanische Schraube (30, 130) mit einer Totpunktkehle (34, 134) versehen ist, die im distalen Teil des Gewindes (32, 132) der mechanischen Schraube derart angeordnet ist, daß eine Drehung der mechanischen Schraube in bezug auf den Körper oder umgekehrt ohne Hervorrufen einer zusätzlichen Verschiebung der biologischen Schraube in bezug auf den Körper ermöglicht wird, wenn sich die Nase in besagter Totpunktkehle befindet.

2. Elektrode nach Anspruch 1, dadurch gekennzeichnet, daß sie eine stationäre Dichtung (40, 140) umfaßt, die einen Elastomerring (44, 144) umfaßt, der im proximalen Bereich des Körpers (10, 110) dieser Elektrode montiert ist und in diesem durch eine Scheibe (50) kraftschlüssig blockiert wird.

3. Elektrode nach Anspruch 1 oder 2, dadurch gekennzeichnet, daß die Dichtung (40) distal Scheiben (48, 148, 151) umfaßt, die in Anlage an die Schulter einer Erweiterungsbohrung (42) des Körpers (10) der Elektrode blockiert ist.

4. Elektrode nach Anspruch 3, dadurch gekennzeichnet, daß es vorgesehen ist, Federn (52, 152, 153) zwischen dem proximalen Ende der mechanischen Schraube (30, 130) und den Scheiben (48, 148, 151), die distal zur stationären Dichtung (40, 140) angeordnet sind, anzuordnen.

5. Elektrode nach einem der vorhergehenden Ansprüche, dadurch gekennzeichnet, daß die mechanische Schraube (30, 130) eine Hülse (58, 158) umfaßt, die im Innern koaxial montiert am distalen Ende besagter Schraube eine Schulter bildet, wobei das proximale Ende der Hülse oder die Verlängerung der mechanischen Schraube die Zentrierung von Federn (52, 152, 153) ermöglicht, während der distale Teil der Hülse zur Zusammenarbeit mit einem Betätigungskabel vorgesehen ist.

6. Elektrode nach Anspruch 1 und 5, dadurch gekennzeichnet, daß das distale Ende der biologischen Schraube eine blockierenden Einschnürung (68, 168) im Inneren der Hülse (58, 158) zur Verwirklichung einer Anflachung (70, 170) umfaßt.

7. Elektrode nach einem der vorhergehenden Ansprüche, dadurch gekennzeichnet, daß das freie distale Ende der Hülse (58, 158), das zur Aufnahme des freien Endes des Betätigungskabels vorgesehen ist, gleichzeitig abgeflacht ist.

8. Elektrode nach einem der vorhergehenden Ansprüche, dadurch gekennzeichnet, daß ein leitendes Kabel (14, 114) vom Typ einer Feder mit aneinanderliegenden Spiralwindungen am distalen Ende des Körpers der Elektrode befestigt ist.

9. Elektrode nach einem der vorhergehenden Ansprüche, dadurch gekennzeichnet, daß die innere Schraube (30, 130) mit einem Gewinde (32, 132) versehen ist und mit der Nase (36, 136) zusammenarbeitet, die ins Innere des Körpers der Elektrode, der das Innengewinde bildet, vorspringt.

10. Elektrode nach einem der vorhergehenden Ansprüche, dadurch gekennzeichnet, daß die Nase (36, 136) eine geneigte Achse (38) und im wesentlichen entsprechend einer Sehne der inneren zylindrischen Ausnehmung des Körpers (10) der Elektrode angeordnet oder eine Verformung (136), die aus dem Körper (110) selbst herauskommt, ist.
